(19) **Europäisches Patentamt** / **European Patent Office** / **Office européen des brevets**

(11) **EP 1 424 064 B1**

(12) **FASCICULE DE BREVET EUROPEEN**

(45) Date de publication et mention de la délivrance du brevet:
**06.06.2007 Bulletin 2007/23**

(51) Int Cl.:
*A61K 8/55* (2006.01)      *A61K 8/49* (2006.01)
*A61Q 19/00* (2006.01)      *A61Q 19/08* (2006.01)

(21) Numéro de dépôt: **03292633.9**

(22) Date de dépôt: **22.10.2003**

(54) **Utilisation de l'adénosine ou d'un analogue d'adénosine, pour lisser les rides d'expression**

Verwendung von Adenosin oder einem Adenosinanalogen zur Glättung von feinen Linien

Use of adenosin or an adenosin analog to smooth out fine lines

(84) Etats contractants désignés:
**AT BE BG CH CY CZ DE DK EE ES FI FR GB GR HU IE IT LI LU MC NL PT RO SE SI SK TR**

(30) Priorité: **26.11.2002 FR 0214828**

(43) Date de publication de la demande:
**02.06.2004 Bulletin 2004/23**

(73) Titulaire: **L'ORÉAL**
**75008 Paris (FR)**

(72) Inventeur: **Galey, Jean-Baptiste**
**93600 Aulnay-Sous-Bois (FR)**

(74) Mandataire: **Renard, Emmanuelle**
**L'OREAL - D.I.P.I.**
**25-29 Quai Aulagnier**
**92600 Asnières (FR)**

(56) Documents cités:
**EP-A- 0 704 210        EP-A- 1 405 633**
**WO-A-01/43704         DE-A- 19 545 107**
**DE-U- 20 022 691       FR-A- 2 746 641**
**FR-A- 2 793 681        FR-A- 2 798 590**
**US-A- 3 978 213        US-A- 5 371 089**
**US-A1- 2003 044 439     US-B1- 6 423 327**

• **DATABASE MEDLINE [Online] US NATIONAL LIBRARY OF MEDICINE (NLM), BETHESDA, MD, US; XP002252592 accession no. STN Database accession no. NLM1653549**

**Description**

La présente invention concerne un procédé cosmétique selon la revendication 1 pour détendre les traits et/ou décontracter la peau, comprenant l'application topique sur la peau d'une composition renfermant, dans un milieu physiologiquement acceptable, au moins un composé choisi parmi l'adénosine et un analogue d'adénosine selon la revendication 1.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané ou en prévenant sa dégradation.

Bien que ces traitements permettent d'agir sur les rides et ridules dues au vieillissement chronologique ou intrinsèque, ainsi que de celles dues au photo-vieillissement, ils n'ont pas d'effet sur les rides d'expression.

Les rides d'expression sont en effet la résultante de mécanismes différents de ceux générant les rides dues au vieillissement.

Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. L'âge, de même que certains facteurs environnementaux tels que l'exposition au soleil, n'intervient pas dans leur genèse mais peut les creuser davantage et les rendre permanentes.

Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

Jusqu'à présent, les seuls moyens couramment utilisés pour agir sur les rides d'expression sont, d'une part, la toxine botulique qui est notamment injectée dans les rides de la glabelle (voir J.D. Carruthers et al., J. Dermatol. Surg. Oncol., 1992, 18, pp. 17-21) et, d'autre part, des implants dégradables à base de collagène, d'acide hyaluronique ou d'acide polylactique.

En outre, comme alternative à ces techniques médicales nécessitant le recours à un praticien, la Demanderesse a proposé divers composés susceptibles d'offrir un effet myorelaxant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques (FR-2 793 681), et en particulier le manganèse et ses sels (FR-2 809 005) et l'alvérine (FR-2 798 590) ; et les agonistes des récepteurs associés aux canaux chlore, dont la glycine (EP-0 704 210) et certains extraits d'*Iris pallida* (FR-2 746 641).

Il reste toutefois le besoin de disposer de composés efficaces pour décontracter la peau en vue de lisser ou d'estomper les rides d'expression.

Or, la Demanderesse a découvert avec étonnement que l'adénosine et ses analogues permettaient de satisfaire ce besoin. Précisément, il a été démontré que l'adénosine permettait de relaxer ou décontracter les cellules contractiles dermiques qui sont supposées être impliquées dans la genèse des rides d'expression. On pense en effet que le phénotype de certains fibroblastes situés le long des lignes de tension créées sous l'effet des contractions des muscles peauciers lors des mimiques serait progressivement modifié sous l'effet de ces contractions, conférant ainsi à ces fibroblastes des propriétés contractiles particulières. La décontraction de ces cellules permettrait ainsi de lutter contre les rides d'expression.

Dans le domaine pharmaceutique, l'adénosine est administrée par voie orale ou intraveineuse comme vasodilatateur et anti-arythmique.

Dans le domaine cosmétique, il a été suggéré dans les documents US-6,423,327 et US 2003/044439 d'utiliser l'adénosine ou un analogue d'adénosine, dans une composition appliquée topiquement sur la peau, pour améliorer l'état de la peau et en particulier pour lutter contre les rides, le relâchement cutané, la sécheresse de la peau et les taches pigmentaires. Il est indiqué que l'adénosine a pour effet d'augmenter la taille des fibroblastes, ainsi que la synthèse de protéines par les fibroblastes.

Dans le même ordre d'idées, les documents WO 01/43704, US-3,978,213, US-5,371,089, DE-195 45 107 et DE-200 22 691 divulguent des compositions à effet anti-âge comprenant de l'adénosine ou un analogue d'adénosine.

Il n'est pas suggéré dans tous ces documents que l'adénosine puisse avoir un quelconque effet décontractant sur les fibroblastes contractiles.

La présente invention a donc pour objet un procédé cosmétique pour lisser ou estomper les rides d'expression par détente des traits et/ou décontraction de la peau, comprenant l'application topique sur la peau sur les zones du front marquées par lesdites rides d'expression d'une composition renfermant, dans un milieu physiologiquement acceptable, de l'adénosine ou un analogue d'adénosine choisi parmi : la 2'-deoxyadénosine ; la 2',3'-isopropylidene adénosine; la toyocamycine ; la 1-méthyladénosine ; la N-6-méthyladénosine ; l'adénosine N-oxyde; le 6-méthylmercaptopurine riboside ; le 6-chloropurine riboside, le 5'-adénosine monophosphate ; le 5'-adénosine diphosphate; la phénylisopropyladénosine, la 1-méthylisoguanosine, la $N^6$-cyclohexyladénosine, la $N^6$-cyclopentyladénosiné, la 2-chloro-N6-cyclopentyladénosine, la 2-chloroadénosine, la $N^6$-phényladénosine, la 2-phénylaminoadenosine, la MECA, la $N^6$-phénéthyladénosine, la 2-p-(2-carboxy-éthyl) phénéthyl-amino-5'-N-éthylcarboxamido-adénosine, la N-éthylcarboxamido-adénosine, la 5' (N-cyclopropyl)-carboxamidoadénosine, la DPMA et le metrifudil ; l'érythro-9-(2-hydroxy-3-nonyl) adénine et l'iodotubercidine.

L'adénosine est préférée pour une utilisation dans la présente invention. Elle est notamment disponible dans le commerce sous forme de poudre auprès de la société PHARMA WALDHOF.

La composition selon l'invention est destinée à être appliquée sur les zones du front marquées par des rides d'expression.

Les rides concernées sont de préférence choisies parmi les rides inter-sourcillières et les rides du front.

La quantité d'adénosine ou analogue utilisable selon l'invention est bien entendu fonction de l'effet recherché et peut donc varier dans une large mesure. Pour donner un ordre de grandeur, la composition utilisée selon l'invention peut comprendre de 0,001 à 10% en poids, de préférence de 0,01 à 1% en poids d'adénosine ou analogue, par rapport au poids total de la composition.

La composition selon l'invention est adaptée à une application topique sur la peau et elle contient donc un milieu physiologiquement acceptable, c'est-à-dire compatible avec la peau.

Cette composition peut être plus ou moins fluide et avoir l'aspect d'une crème blanche ou colorée, d'une pommade, d'un lait, d'une lotion, d'un sérum, d'une pâte, d'une mousse. Elle peut également se présenter sous forme solide, en particulier sous forme de stick. Elle peut être utilisée comme produit de soin et/ou comme produit de maquillage pour la peau.

La composition selon l'invention peut se présenter sous toutes les formes galéniques normalement utilisées dans le domaine cosmétique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les hydrocarbures d'origine minérale ou synthétique (huile de vaseline, isohexadécane), les huiles d'origine végétale (huile d'amande d'abricot, fraction liquide de beurre de karité, d'avocat, huile de soja), les huiles d'origine animale (lanoline), les huiles de synthèse (perhydrosqualène, tétraoctanoate de pentaérythrityle), les huiles siliconées (cyclopentasiloxane et cyclohexasiloxane) et les huiles fluorées (perfluoropolyéthers). On peut aussi utiliser comme matières grasses des alcools gras (alcool cétylique ou stéarylique), des acides gras (acide stéarique), des cires (cire de carnauba, ozokérite, cire d'abeille).

Comme émulsionnants et coémulsionnants utilisables dans l'invention, on peut citer par exemple les esters d'acide gras et de polyéthylène glycol tels que le stéarate de PEG-100 et le stéarate de PEG-20 et les esters d'acide gras et de glycérine tels que le stéarate de glycéryle.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées de l'adénosine.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme conservateurs, on peut citer les esters d'acide para-hydroxybenzoïque, l'octane 1,2-diol, l'iodo-3 propynyl-2 butyl carbamate, le phénoxyéthanol et le gluconate de chlorhexidine.

Comme charges, on peut citer par exemple, les particules de polyamide (Nylon) ; les microsphères de polyméthacrylate de méthyle ; les poudres de copolymère éthylèneacrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

Comme indiqué précédemment, la composition selon l'invention peut également renfermer des filtres UVA et/ou UVB, sous forme de composés organiques ou inorganiques, ces derniers étant éventuellement enrobés pour les rendre hydrophobes.

Les filtres organiques plus particulièrement préférés sont choisis parmi les composés suivants (cités selon la nomenclature CTFA) : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyte)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les filtres inorganiques sont de préférence constitués d'oxyde de zinc, de fer, de zirconium, de cérium et/ou de titane (amorphe ou cristallisé sous forme rutile et/ou anatase), de préférence de taille nanométrique (taille moyenne des particules primaires: généralement entre 5 nm et 100 nm, de préférence entre 10 nm et 50 nm), éventuellement enrobés d'alumine et/ou d'acide stéarique.

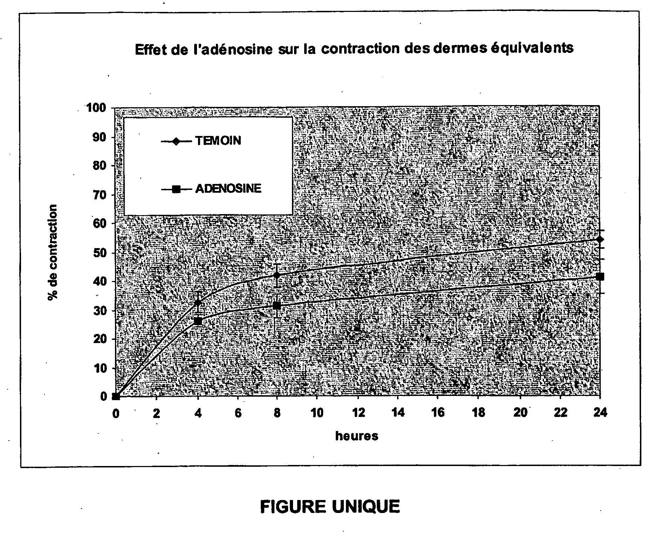

L'invention sera maintenant illustrée par les exemples suivants. Dans ces exemples, il est fait référence à la Figure annexée qui illustre la contraction au cours du temps d'une derme équivalent traité par l'adénosine.

## EXEMPLES

### Exemple 1 : Mise en évidence de l'effet dermo-décontractant de l'adénosine

a) Principe du test

Le principe de ce test a consisté à étudier l'effet relaxant de l'adénosine sur un modèle de derme équivalent constitué d'une matrice de collagène ensemencée par des fibroblastes humains normaux.

Ces conditions sont destinées à mimer *in vitro* les phénomènes contractiles dermiques qui se produisent lors des mimiques du visage. Dans ces conditions, en effet, les cellules expriment spontanément des forces de traction qui induisent une rétraction du gel de collagène. Il en résulte une diminution de la surface totale du derme équivalent au cours du temps. La mesure de cette surface permet d'évaluer les effets de relaxation des substances préalablement mises en contact avec le derme équivalent.

b) Protocole

Deux séries de 3 dermes équivalents attachés contenant des fibroblastes humains normaux sont préparés : une série témoin sans aucun traitement, et une série traitée par l'adénosine (0,01 %). L'expérience est répétée trois fois.

Les équivalents de derme sont préparés comme décrits dans Asselineau et col., Exp. Cell. Res., 1985, 159, 536-539 ; Models in dermatology, 1987, vol 3 pp 1-7, dans les proportions suivantes :

| | |
|---|---|
| Milieu MEM (1,76X) avec ou sans adénosine | 45% |
| Sérum de Veau Foetal : | 9% |
| NaOH (0.1N) : | 5% |
| Acide acétique (1/1000) : | 4% |
| Collagène : | 26% |
| Fibroblastes : | 11% |

Le derme équivalent traité diffère du derme équivalent témoin en ce que l'on y ajoute 0,01% d'adénosine.

Le collagène utilisé est du collagène de type 1 (solution commerciale), mais on peut également utiliser du collagène de type III ou IV. Il est extrait de queues de rat ou de peau de veau par hydrolyse acide et conservé en milieu acide à +4°C ; il polymérise naturellement par réchauffement à 37°C et par diminution du taux d'acidité. Le collagène est préalablement dialysé contre des bains successifs d'eau + acide acétique.

Le protocole est le suivant : dans un tube flacon stérile, on introduit le milieu MEM 1,76 X en présence d'additifs (Glutamine 1%, Acides aminés non essentiels 1%, Pyruvate de sodium 1%, Fungizone 1% et Pénicilline/Streptomycine 1%), le sérum de veau foetal, la soude NaOH 0,1 N. On ajoute alors les fibroblastes isolés à partir d'explants de peau humaine à la concentration de 1,4x $10^5$ cellules pour 1 ml de milieu de culture.

On ajoute alors lentement, contre la paroi du tube de façon à observer l'apparition d'un nuage blanchâtre, un mélange volume/volume de collagène dans de l'acide acétique au 1/1000.

L'ensemble est alors mélangé avec précaution et réparti dans les puits d'une plaque de culture de 12 puits (type Costar référence 3512) à raison de 0.5ml de mélange par $cm^2$. La plaque de culture est alors placée dans un incubateur à 37°C avec 5% de $CO_2$.

Une fois formés après polymérisation du collagène, les dermes équivalents sont laissés adhérents au support de culture pendant 3 jours puis détachés du support pour que la contraction puisse démarrer. Ces dermes équivalents attachés sont sortis de l'incubateur pour effectuer les prises d'image en vue de la mesure de leur surface et ce pour chaque point de la cinétique de contraction (0, 4, 8 et 24 heures). Ils sont immédiatement remis dans l'incubateur entre chaque point de mesure.

L'évaluation de la contraction spontanée des dermes équivalents traité (avec adénosine) et témoin (sans adénosine) est réalisée en mesurant leur surface, à différents temps après le début de la contraction spontanée.

Pour cela, une image numérique est acquise pour chaque derme équivalent traité ou non traité au moyen d'une caméra (Caméra CCD -Iris Sony DXC -107P) et la surface est ensuite calculée sur chaque image au moyen d'un système d'analyse d'image (Zeiss Axiovision 3.0). A cette mesure de surface correspond un pourcentage de contraction égal au rapport des surfaces selon la formule :

$$\% \text{ contraction} = (Sp-Si)/Sp \times 100$$

où :

'Sp' représente la surface d'un puits de la plaque de culture ; elle correspond à la surface totale du derme équivalent avant contraction

'Si' représente la surface du derme équivalent à l'instant i de la cinétique de contraction.

c) Résultats

Comme illustré à la Figure ci-jointe, le taux de contraction du derme équivalent témoin est de 32% quatre heures après l'avoir détaché de son support. Il progresse à 42% après huit heures pour atteindre 54% après vingt-quatre heures.

L'adénosine réduit ce pourcentage de contraction de 6,4% après quatre heures, 10,5% après huit heures et 12,7% après vingt-quatre heures, par rapport au témoin.

Ce test démontre ainsi que l'adénosine entraîne une moindre contraction du derme équivalent, donc un effet relaxant qui peut être mis à profit pour la préparation de compositions cosmétiques à effet dermo-décontractant.

**Exemple 2 : Composition cosmétique**

Cette composition est préparée de manière classique pour l'homme du métier. Les quantités données dans cet exemple sont indiquées en pourcentages pondéraux.

| | |
|---|---|
| Adénosine | 0,10% |
| Acide stéarique | 3,00% |
| Mélange de mono-stéarate de glycéryle et stéarate de polyéthylène glycol (100 OE) | 2,50% |
| Stéarate de polyéthylène glycol (20 OE) | 1,00% |
| Cyclopentadiméthylsiloxane | 10,00% |
| Charges | 3,00% |
| Huiles végétales | 7,00% |
| Huiles synthétiques | 6,00% |
| Conservateurs | 1,20% |

(suite)

| Diméthylsiloxane oxyéthyléné (16 OE) à extrémités méthoxy | 1,00% |
| Gomme de silicone | 0,20% |
| Copolymère acrylique en émulsion inverse (Simulgel 600 de SEPPIC) | 1,70% |
| Alcool stéarylique | 1,00% |
| Eau | qsp 100 % |

Cette crème est destinée à être appliquée sur le visage et le front pour détendre les traits et décontracter la peau du visage.

## Revendications

1. Procédé cosmétique pour lisser ou estomper les rides d'expression par détente des traits et/ou décontraction de la peau, comprenant l'application topique sur la peau sur les zones du front marquées par lesdites rides d'expression d'une composition renfermant, dans un milieu physiologiquement acceptable, de l'adénosine ou un analogue d'adénosine choisi parmi : la 2'-deoxyadénosine ; la 2',3'-isopropylidene adénosine; la toyocamycine ; la 1-méthyladénosine ; la N-6-méthyladénosine ; l'adénosine N-oxyde; le 6-méthylmercaptopurine riboside ; le 6-chloropurine riboside, le 5'-adénosine monophosphate ; le 5'-adénosine diphosphate; la phénylisopropyladénosine, la 1-méthylisoguanosine, la $N^6$-cyclohexyladénosine, la $N^6$-cyclopentyladénosine, la 2-chloro-N6-cyclopentyladénosine, la 2-chloroadénosine, la $N^6$-phényladénosine, la 2-phénylaminoadénosine, la MECA, la $N^6$-phénéthyladénosine, la 2-p-(2-carboxy-éthyl) phénéthyl-amino-5'-N-éthylcarboxamido-adénosine, la N-éthylcarboxamido-adénosine, la 5' (N-cyclopropyl)-carboxamidoadénosine, la DPMA et le metrifudil ; l'érythro-9-(2-hydroxy-3-nonyl) adénine et l'iodotubercidine.

2. Procédé selon la revendication 1, **caractérisé en ce que** les rides d'expression sont choisies parmi les rides inter-sourcillières et les rides du front.

3. Procédé selon l'une des revendications 1 ou 2, **caractérisé en ce que** la composition comprend de 0,01 à 1% en poids d'adénosine ou analogue, par rapport au poids total de la composition..

## Claims

1. Cosmetic process for smoothing or fading out expression wrinkles by relaxing the features and/or decontracting the skin, comprising the topical application, to the skin on the areas of the forehead marked by said expression wrinkles, of a composition containing, in a physiologically acceptable medium, adenosine or an adenosine analogue chosen from: 2'-deoxyadenosine; 2',3'-isopropylidene adenosine; toyocamycin; 1-methyladenosine; N-6-methyladenosine; adenosine N-oxide; 6-methylmercaptopurine riboside; 6-chloropurine riboside; 5' -adenosine monophosphate; 5'-adenosine diphosphate; phenylisopropyladenosine, 1-methylisoguanosine, $N^6$-cyclohexyladenosine, $N^6$-cyclopentyladenosine, 2-chloro-$N^6$-cyclopentyladenosine, 2-chloroadenosine, $N^6$-phenyladenosine, 2-phenylaminoadenosine, MECA, $N^6$-phenethyladenosine, 2-p- (2-carboxyethyl)phenethylamino-5'-N-ethylcarboxamidoadenosine, N-ethylcarboxamidoadenosine, 5'-(N-cyclopropyl)carboxamidoadenosine, DPMA and metrifudil; erythro-9- (2-hydroxy-3-nonyl)adenine and iodotubercidine.

2. Process according to Claim 1, **characterized in that** the expression wrinkles are chosen from the wrinkles between the eyebrows and the wrinkles on the forehead.

3. Process according to either of Claims 1 and 2, **characterized in that** the composition comprises from 0.01% to 1% by weight of adenosine or analogue relative to the total weight of the composition.

**Patentansprüche**

1. Kosmetisches Verfahren zum Glätten oder Kaschieren von Mimikfalten durch die Lockerung der Züge und/oder die Entspannung der Haut, das das topische Auftragen einer Zusammensetzung auf die Haut in den Bereichen der Stirn beinhaltet, die von diesen Mimikfalten gezeichnet sind, die sie in einem physiologisch akzeptablen Medium Adenosin oder ein Adenosinanalogon enthält, das ausgewählt ist unter: 2'-Desoxyadenosin; 2',3'-Isopropylidenadenosin; Toyocamycin; 1-Methyladenosin; N6-Methyladenosin; Adenosin-N-oxid; 6-Methylmercaptopurinribosid; 6-Chlorpurinribosid; 5'-Adenosinmonophosphat; 5'-Adenosindiphosphat; Phenylisopropyladenosin, 1-Methylisogua-nosin, $N^6$-Cyclohexyladenosin, $N^6$-Cyclopentyladenosin, 2-Chlor-N6-cyclopentyladenosin, 2-Chloradenosin, $N^6$-Phenyladenosin, 2-Phenylaminoadenosin, MECA, $N^6$-Phenethyladenosin, 2-p-(2-Carboxy-ethyl)-phenethylamino-5'-N-ethylcarboxamidoadenosin, N-Ethylcarboxamidoadenosin, 5'-(N-cyclopropyl)-carboxamidoadenosin, DPMA und Metrifudil; Erythro-9-(2-hydroxy-3-nonyl)-adenin oder Iodtubercidin.

2. Verfahren nach Anspruch 1, **dadurch gekennzeichnet, dass** die Mimikfalten unter den Falten zwischen den Augenbrauen und den Stirnfalten ausgewählt sind.

3. Verfahren nach einem der Ansprüche 1 oder 2, **dadurch gekennzeichnet, dass** die Zusammensetzung 0,01 bis 1 Gew.-% Adenosin oder Adenosinanalogon, bezogen auf das Gesamtgewicht der Zusammensetzung, enthält.

Effet de l'adénosine sur la contraction des dermes équivalents

**FIGURE UNIQUE**